# EUROPEAN PATENT SPECIFICATION

(11) **EP 0 425 696 B1**
(45) Date of publication and mention of the grant of the patent: **12.07.1995**
(21) Application number: 90907418.9
(22) Date of filing: 09.05.1990
(51) Int. Cl.: A61M 25/10

(54) **CATHETER-CUM-BALLOON**
BALLONKATHETER
CATHETER MUNI D'UN BALLON

(30) Priority: 09.05.1989 JP 116607/89
(43) Date of publication of application: 08.05.1991
(73) Proprietor: TORAY INDUSTRIES, INC., Tokyo 103 (JP)
(72) Inventor: NOGUCHI, Noriyasu, Shiga 520-23 (JP); YAMAZAKI, Yoshiharu, Shiga 520 (JP); HAGIO, Mitsuyuki, Kanagawa 248 (JP); FUTAMI, Yasuhiko, Tokyo 158 (JP)
(74) Representative: Kador & Partner
(86) International application number: PCT/JP90/00587
(87) International publication number: WO 90/13331

(56) References cited:
- EP-A- 0 186 267
- GB-A- 1 566 674
- JP-A-61 228 877
- US-A- 3 657 873
- US-A- 4 121 408

## Description

The present invention relates to a catheter with a balloon for expanding a constricted valve of the heart or a constricted or occluded blood vessel and normalizing thereby blood flow in medical treatment.

As material for the balloon, those thin membranes (films) of synthetic resins such as polyethylene, polyvinyl chloride or polyester which have no stretchability but pressure resistance have been used for preventing blood vessels from damage caused by an excessive expansion, it is not preferable that when these balloons are expanded, the surfaces thereof become to be slippery to cause the balloons to slip out or when they are shrunk, a lot of wrinkles are thereon produced and they rub the inner walls of blood vessels to give them damages and to accelerate formation of thrombi.

Therefore, it has been originated that such an improvement as not to form wrinkles when shrunk performed by using a polyurethane or a silicone as a material for the balloon (Japanese Laid-Open Patent No.91970/1984 and No.103,453/1986) or drawing the shrunk balloon (Japanese Laid-Open Patent No.125,386/1978), or an excessive expansion was prevented and the expansion was made uniform by incorporating a knitted fabric into a balloon.

In addition, Inoue originated a design wherein a rubber as a balloon raw material and a guitar-shaped constricted part on the balloon by pinching it with a knitted fabric with several stripes of bands was made and a constricted valve of the heart was pinched therewith and the balloon was not slipped out (Japanese Laid-Open Patent No. 23506/1982).

However, even when these balloons were used, the outer diameters became too large from their structural points and exposure and incision of a blood vessel were required when the balloon was inserted into them. It resulted in that it took longer time for recovery and it gave a life of a patient inconvenience after medical treatment.

In EP-A-0 186 267 a catheter is described having a balloon that is reinforced with a composite yarn. This composite yarn is consisting of an elastic yarn and a non-elastic yarn having a larger free length than the elastic yarn. The composite yarn may form a cylindrical fabric disposed between an outer elastic film and in inner elastic film of the balloon. The fabric is prepared by a yarn strand consisting of two parallel twisted plies which, however, revealed to easily separate during their processing.

The problems of the conventional technologies, are solved by a balloon catheter as defined in claims 1 and 9. Preferred embodiments are given in subclaims 2 to 8.

As the catheter with a balloon of the present invention exhibits larger balloon stretchability, it is possible to make the outer diameter smaller for a balloon with large expansion diameter used in a tearing and opening technique for a valve of the heart etc., and the catheter with the balloon can be inserted in a blood vessel by means of a simple Celludinga method. Therefore, there exist such advantages that a cut wound for a surginal operation could be smaller; treatment and management after the surginal operation are simple and the time required for recovery is extremely short; smaller daily burdens of a patent and an attendant are needed.

In addition, when the catheter with a balloon of the present invention is pushed into a blood vessel and an inner heart to a diseased part, an inserting operation putting a guide wire and an expanded balloon in the blood flow can be easily performed and as the apex of the balloon is soft, there is few possibility of damaging the inner wall of the blood vessel and the workability thereof is also good. Moreover, preparation of a cylindrical body made of a composite yarn and a cylindrical elastic film and assembling them for manufacturing the balloon are easy and the yield is good. The cost can be towered, too.

### Brief Description of the Drawings

An example of a structure of a catheter with a balloon of the present invention will be explained by utilizing drawings.

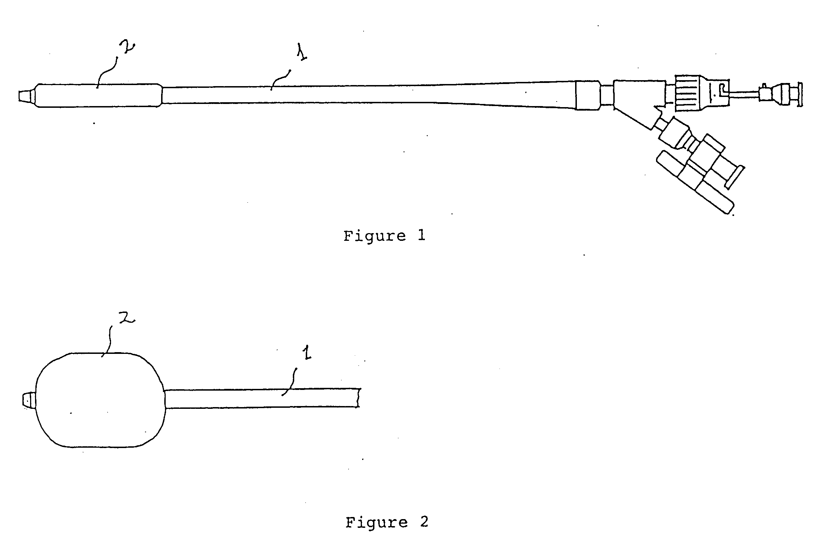

Figure 1 is a rough figure of a catheter with a balloon of the present invention and Figure 2 is a figure illustrating an expanded state of the balloon part of the catheter with the balloon in Figure 1.
- 1:: A tube part
- 2:: A balloon part

### Best Embodiment for Practicing the Present Invention

As illustrated in Figure 1, in the catheter with a balloon 2 constituting a three-layered structure with a cylindrical body consisting of a cylindrical elastic film and a composite yarn is attached on the apex of a tube 1 with a smooth surface and a round cross-section and on the rear end of the tube 1, if necessary, a fluid inlet and an take-up opening for an air removal tube placed in a fluid transferring path are provided. In the catheter with a balloon of the present invention, the ratio of the free length of an elastic yarn to a non-elastic yarn is preferably 0.15-0.5 and more preferably 0.2-0.35. If this value is too large, the balloon hardly becomes to be sufficiently expanded, and if this value is too small, a reinforcing effect by means of the non-elastic yarn is easily fluctuated and breakage of the balloon when expanded easily occurs. However, if a careful consideration is payed for manufacturing the balloon to avoid the breakage, even when said ratio is larger than the above described value, the effect of the present invention can be utilized. Said ratio of the free length can be known by measuring a kink of the S-S curve of said composite yarn to the higher tension side. When the free length of the non-elastic yarn is fluctuated and said kink is therefore unclear, it can be known by extrapolating the straight lines before and behind the point.

As the structure of said composite yarn, a core-sheath yarn wherein the core is made of an elastic yarn and the sheath is made of a non-elastic yarn is preferable because both yarns are hardly separated.

Thus, to make it easy to stretch the balloon by an inner pressure or to fix the order of stretching of each portion of the balloon or to fix the final stretched shape, in some cases it is necessary to form the habit of order or the shape to the balloon by repeated stretching and shrinking. In such cases, separation of the two constituent yarns may cause unfavourable effects.

As the method for manufacturing the core-sheath yarn, a method wherein twisting or entangling by means of a fluid is performed while a non-elastic yarn is overfed is preferable and a core-sheath yarn made by winding spirally a twisted yarn of a synthetic fiber on a urethane yarn is especially preferable as it is compact. As the non-elastic yarn, a textured yarn is preferable as its stretchability, adhesiveness with an elastic film etc., are excellent. Among textured yarns, false-twisted textured yarns with excellent stretchability are preferable. When a non-textured yarn is used, there exists a defect that separation thereof from an elastic yarn easily occurs but said defect can be avoided by taking such countermeasures as increasing sufficiently the number of twists.

It is preferable that the balloon consists of a three-layered structure of an elastic film/a cylindrical body of a composite yarn/an elastic film. Namely, the inner elastic film provides an action utilizing a reinforcing effect caused by the composite yarn and the outer elastic film provides an action making passing through the blood vessel better. The balloon based on the present invention can be applied to most catheters with a balloon, but a furthermore excellent effect can be expected when it is applied to a catheter with a balloon wherein one end of the balloon is fixed on the inner tube (the inner tube may be an inner-filled rod) and another end thereof is fixed on the outer tube of a double tube wherein both inner and outer tubes can be concentrically slidable each other. Namely, in said catheter with a balloon, the outer diameter of the balloon can be furthermore decreased by sliding both tubes and thereby stretching the balloon in the axial direction when it is inserted in a blood vessel and inside of the heart or taken out thereof. If the rigidity of the inner tube is too small not to stretch the balloon, the balloon can be effectively stretched in the axial direction by providing a stopper on the apex of said inner tube and pushing it with a rod insertable in said inner tube. If the rigidity of the outer tube is insufficient, it is preferable that a high-modules fiber is fixed on the apex of the outer tube and it is guided to the opposite end.

The elastic film is often made of a thin membrane with large stretchability and smooth surface in such a way that the thickness of the front end side is thinner than that of the rear end. As the raw material, polyurethanes and rubbers are used and especially, rubbers having small initial moduli are excellent materials as raw materials for the elastic film.

The thickness of the elastic film is preferably in the range of 0.1-0.4 mm and it is not necessarily the same thickness between the inner and the outer layers.

Difference in thickness between the front end side and the rear end side is preferably about 5-20% and the difference can be obtained by making it thinner, for example, when a cylindrical elastic film is prepared or by expanding a corresponding part of the prepared cylindrical elastic film and fixing the residual elongation thereof as a simple method.

As the cylindrical body made of the composite yarn, a knitted fabric, a woven fabric, a braid or a wound body in a cylindrical shape with a large twill angle can be used and among them, a cylindrically-knitted structure with stretchability is preferable.

When a guitar-shape balloon is prepared, for example, an elastic band are adhered on the middle part of an elastic body with an adhesive such as a rubber paste.

As the elastic yarn being a core yarn for a textured yarn, any fiber with stretchability can be used without specific limitation. For example, a single yarn or a twisted yarn made of a rubber such as natural rubbers and synthetic rubbers or a polyurethane is suitable and as the yarn to be wound made of a synthetic fiber, not only high-tenacity nylon, polyester and "Teflon" yarns but high-tenacity polyimide and polyethylene yarns can be used as a single yarn, but a twisted yarn being flexible is preferable. The thicknesses of these yarns are not specifically limited as the required pressure-resistant characteristics are different depending upon the position to be worked and the purpose, but in many cases, the thickness of the elastic yarn being a core yarn is selected in the range of 11-56 dtex (10-50D) and that of the tenacity or high tenacity yarn to be wound is selected in the range of 33-167 dtex (30-150D).

Then, if the outer elastic film is adhered on a cylindrical body of a composite yarn and the inside of the cylindrical body of the composite yarn between an elastic film is coated with a silicone oil etc., a gap caused by expansion or shrinkage is not produced and a uniform expansion can be obtained. The length of the balloon can be freely set and in many cases, it is preferably in the range of 15-70 mm.

It is preferable that both ends and apexes of a balloon and the connected parts with tubes can be finished with an adhesive to make their surfaces smooth.

As the raw material used for a soft inner tube in coaxially (concentrically)-assembled tubes, for example, polyvinyl chloride and polyurethane are preferably used. The length of this tube is preferably 5-25 cm and it is preferable that the diameter is 4,7 mm (14 Fr) or smaller as it is inserted by means of Celludinga method.

In addition, as the relatively hard outer tube with torque characteristics, for example, a tube made of a synthetic resin such as polyethylene, "Teflon", polypropylene and nylon is used. Besides these tubes, for example, hard tubes such as a tube made by extracting a plasticizer for polyvinyl chloride with a solvent or a tube cured by crosslinking by means of irradiation. It is preferable that the diameter of this outer tube is 4,7 mm (14 Fr) or smaller because of the above described reason. In addition, the length is not specifically defined as it is different depending on age and size of the patient and the distance from the inserted part to the position to be cured, but a product with a total length in the range of 50-200 cm is preferably used.

In addition, depending on the position to be cured, a relatively hard tube similar to the outer tube can be sometimes preferably substituted for the soft inner tube as the relatively hard tube provides an action making insertion of a rigid body easy.

Furthermore, if a contrast-medium such as barium sulfate, bismuth compd. or a metal powder such as tungsten is mixed in these tubes, it is preferable that the position in the blood vessel can be helpfully thereby detected by X-ray irradiation.

In addition, as the raw material of the yarn or the knitted fabric buried in or set along with the soft inner tube, a thin and strong one is preferable and for example, said yarns can be all used but polyamide yarns are the best among them.

In addition, it is necessary that an air removal tube set between an inner tube and an outer tube does not interference the flexibility of the soft tube and a tube with a thin thickness and a small diameter, for example, a tube made of polyimide or "Teflon" or a polyethylene tube is used.

As a rigid body inserted in an inner tube and stretching a balloon, for example, a stainless pipe or thin rod can be used.

In addition, as a fluid expanding a balloon, for example, physiological saline, carbon dioxide or a contrast-medium is used. These fluids are used by a method wherein a specified amt. thereof is injected thereinto by means of an injection cylinder etc.

The catheter with a balloon of the present invention has such features that the stretchability is large and it exhibits pressure resistance and a uniform expanding property by using a cylindrical body made of a composite yarn with enlarged stretchability.

By the present invention, even a balloon with a large diameter can be inserted in a blood vessel in such a way that it is made long and slender and small and in addition, there is no possibility of damaging the inner wall of the blood vessel when the apex part of a tube rubs it and the balloon can be transferred to the position to be cured through the blood vessel and the heart by utilizing a guide wire and the floating property of the balloon.

Therefore, the time required for curing is short and management after surginal operation is simple and recovery can be rapidly performed. Furthermore, this method can be provided as a method wherein expansion of the occluded part of a blood vessel or an opening technique for a valve of the heart by means of a balloon can be non-operationally, simply and easily performed. In addition, the balloons of the present invention can be furthermore simply manufactured in such a way that products with various sizes are separately manufactured to provide catheter with balloons with good yield and with low cost.

### (Examples)

The present invention will be explained in more detail by using examples as follows.

### Example 1.

A false-twisted textured yarn consisting of a polyester yarn (a non-elastic yarn) of 78 dtex (70D) was inter-twisted to a polyurethane yarn (an elastic yarn: "Opelon") of 22 dtex (20D) at a speed of 400 T/m (twist number in turn per meter) while the former was overfed 300% to said urethane yarn to manufacture a composite yarn.

The ratio of free length of said yarn was 0.26 read from the S-S curve.

A balloon was prepared by using this composite yarn.

Namely, as an outer tube, a polyethylene tube containing a contrast-medium with 13.5 Fr and a length of 560 mm and a polyvinyl chloride tube containing 45 parts of DOP with 4,5 mm (13.5 Fr) and a length of 130 mm were connected through a stainless pipe with a diameter of 3.5 mm and a stainless pipe with a diameter of 3 mm and a length of 6 mm was attached on the apex of the polyvinyl chloride tube. In this time, a polyimide twisted yarn of 444 dtex (400D) was placed on the hollow side of the polyvinyl chloride and fixed on stainless pipes on both sides. As an inner tube, on the apex of a polyvinyl chloride tube containing 45 parts of DOP with 2,2 mm (6.6 Fr) a stainless pipe with a diameter of 1.6 mm and a length of 7 mm having a projected part inside made by means of a corset was attached and a stainless pipe with 0,21 mm (outer diameter) (14G) and a length of 70 mm and a needle base was inserted in the rear end. Connection of the inner and outer pipes and the inserted stainless pipe were bound and fixed with a nylon yarn with a thickness of 70 »m. This inner tube was passed through a W-connector and furthermore inserted into the outer tube and then said outer tube was fixed on the W-connector to prepare a tube part.

Then, as an inner elastic film of the balloon, a rubber with a thickness of 0.25 mm and a length of 25 mm was bound and fixed on each apex of the inner tube and the outer tube with a nylon yarn with a thickness of 70 »m and the surface thereof was coated with a small amount of a silicone oil. Separately, a cylindrically-knitted fabric was prepared by knitting said composite yarn by means of a knitting machine wherein 50 knitting needles were arranged on a circumference with a diameter of 20 mm. On the middle part of said knitted fabric with a length of 30 mm, a rubber with a thickness of 0.25 mm and a length of 7 mm was adhered with a rubber paste to prepare a band. Furthermore on the outside thereof, a part prepared by adhering a rubber tube with a thickness on the front side of 0.25 mm and a thickness on the rear side of 0.3 mm was covered. They were all fitted together in such a way that the total length was 25 mm and both ends thereof were bound and fixed with a nylon yarn with a thickness of 70 »m. Then, excess knitted fabric and rubbers were cut off to complete a balloon part.

Then, after a polyimide tube with a diameter of 0.9 mm was inserted into a fluid transferring path between the inner and outer tubes from one of remaining openings of the W-connector up to 10 mm to the balloon, the rear part up to the inside of the W-connector was covered with a reinforcing polyethylene tube. After a two directional stopcock was fixed on the rear end thereof and the gaps of the openings were filled with an adhesive material, finally, the surfaces of the both ends of the balloon, the apex and the connected parts of tubes were finished smoothly with an epoxy resin to complete a catheter with a balloon of the present invention.

This catheter with a balloon was attached on an injection cylinder and after water was substituted for air while air was evacuated from an air vent, about 7 ml of water was injected therein. Only apex part of this balloon was expanded to about 23 mm. Then, when the injected amount was made to 23 ml, the balloon turned into a cylindrical shape with a diameter of about 28 mm.

Then, water was pulled out and the surface was smoothly shrunk without producing wrinkles. Then, a stainless steel tube with a diameter of 1.19 mm was inserted into the inner tube to push the apex part of the inner tube and to stretch thereby the balloon from 25 mm to 55 mm. The diameter of the balloon was changed thereby from about 7 mm to 4.5 mm. When the stainless tube was pulled backward, the balloon was returned to its original diameter and length.

### Example 2.

A catheter with a balloon similar to the one of Example 1 was used for a clinical treatment for an inter-related tearing and opening technique for the mitral valve. Namely, using a method perforating the atrium partition, a specially-made guide wire was inserted into the left atrium through the skin, the crotch vein, the right atrium and the atrium partition. Then, a dilator of 14 Fr was forwarded along with this guide and the perforated parts on the crotch vein and the atrium partition were expanded. A catheter with a balloon of the present invention was successively inserted into the left atrium from the right atrium through the atrium partition and only the apex of the balloon was expanded to a diameter of 10 mm by using carbon dioxide and it was run into a valve opening with the blood flow as a Swanganz catheter was. Then, it was inserted into the left ventricle and the apex side of the balloon was furthermore expanded by using a contrast-medium and it was lightly drawn nearby and brought into contact with the valve opening to be teared and opened. At this position, the balloon was furthermore expanded and the valve opening was pinched with the constricted balloon. The balloon was expanded as they were under this condition and the valve opening can be enlarged without slipping out of the balloon.

The tearing and opening technique needed 1 hour containing 5 sec for expansion and shrinkage of the balloon without any side effect. 3 days were needed from entering a hospital to leaving the hospital. The time needed was only less than a half in comparison with a tearing and opening technique accompanied with exposure and cutting-out of the blood vessel.

### Example 3.

A catheter with a balloon was prepared in the same way as in Example 1, but the denier of "Opelon" yarn as the elastic yarn was 33 dtex (30D) and the ratio of overfeeding a polyester yarn and the number of twist thereof were changed to 270% and 400 T/m (twist number in turn per meter) respectively.

In this catheter with a balloon, the ratio of free length of the constituting yarns of the composite yarn was apparently 0.28 and the balloon exhibited the maximum expanded diameter of 30 mm and the minimum shrunk diameter of 4-5 mm and excellent stretching characteristics without generation of wrinkle.

### Possibility of Industrial Application

The catheter with a balloon of the present invention can be widely used for a method for medical treatment wherein a shrunk balloon is got into a constricted or occluded blood vessel and a fluid is pressed into the balloon to expand the balloon and the expanded balloon enlarges the constricted or the occluded blood vessel to make the constricted or occluded part normal. Especially, it can be widely used for a non-surgical and through-skin type valve tearing and opening operation substituting for a valve incisive operation for the valve of the heart which is constricted by aging, calcination or a sequela of rheumatic heart and a valve substitutive operation for an artificial valve.

## Claims

1. A catheter with a balloon (2) reinforced with a composite yarn consisting of an elastic yarn and a non-elastic yarn with a larger free length than that of the elastic yarn, **characterized in that**, the composite yarn is a core-sheath yarn wherein the elastic yarn is the core yarn and the non-elastic yarn is the sheath and wherein the ratio of free length of the elastic yarn to the non-elastic yarn is from 0.15 to 0.5.

2. The catheter with a balloon according to claim 1 **characterized in that** the ratio of free length of the elastic yarn to the non-elastic yarn is from 0.2 to 0.35.

3. The catheter with a balloon according to claim 1 **characterized in that** the core-sheath yarn is a core-sheath yarn wherein a synthetic fiber-twisted yarn is spirally wound on a urethane yarn.

4. The catheter with a balloon according to claim 1 **characterized in that** the non-elastic yarn is a false-twisted textured yarn.

5. The catheter with a balloon according to claim 1, **characterized in that**, the composite yarn is knitted or woven to a fabric.

6. The catheter with a balloon according to claim 7 **characterized in that** the fabric is knitted.

7. A catheter with a balloon as described in claim 1 **characterized in that** one end of the balloon is fixed on the inner tube of a double tube, wherein said tubes are concentrically slidable against each other, and another end thereof is fixed on the outer tube.

8. A catheter with a balloon according to claim 7, **characterized by** having a shaft insertable in said inner tube and a stopper for said shaft on the apex of said inner tube.

9. A catheter with a balloon (2) having a three layered structure consisting of an outer elastic film, an inner elastic film and a cylindrical fabric of composite yarn therebetween, **characterized in that** the composite yarn is a core-sheath yarn, wherein the core yarn is an elastic yarn and the sheath yarn is a non-elastic yarn and wherein the ratio of free length of the elastic yarn to the non-elastic yarn is from 0.15 to 0.5 and in that the cylindrical fabric of composite yarn is adhered to the outer film and not to the inner film.

## Patentansprüche

1. Katheter mit Ballon (2), der mit einem Verbundgarn verstärkt ist, das aus einem elastischen Garn und einem nicht elastischen Garn mit einer größeren freien Länge als die des elastischen Garns besteht, dadurch gekennzeichnet, daß das Verbundgarn ein Kern/Hüllen-Garn ist, wobei das elastische Garn das Kerngarn und das nicht elastische Garn die Hülle darstellt, und wobei das Verhältnis der freien Länge des elastischen Garns zum nicht elastischen Garn 0,15 bis 0,5 beträgt.

2. Katheter mit Ballon nach Anspruch 1, dadurch gekennzeichnet, daß das Verhältnis der freien Länge des elastischen Garns zum nicht elastischen Garn 0,2 bis 0,35 beträgt.

3. Katheter mit Ballon nach Anspruch 1, dadurch gekennzeichnet, daß das Kern/Hüllen-Garn ein Kern/Hüllen-Garn ist, bei dem ein Garn aus gedrehter synthetischer Faser spiralförmig auf Urethangarn gewickelt ist.

4. Katheter mit Ballon nach Anspruch 1, dadurch gekennzeichnet, daß das nicht elastische Garn texturiertes Falschdrahtgarn ist.

5. Katheter mit Ballon nach Anspruch 1, dadurch gekennzeichnet, daß das Verbundgarn zu einem Textilerzeugnis gewirkt oder gewebt ist.

6. Katheter mit Ballon nach Anspruch 7, dadurch gekennzeichnet, daß das Textilerzeugnis gewirkt ist.

7. Katheter mit Ballon nach Anspruch 1, dadurch gekennzeichnet, daß ein Ende des Ballons am Innenrohr eines Doppelrohrs angebracht ist, wobei die Rohre konzentrisch gegeneinander gleiten können, und das andere Ende am Außenrohr angebracht ist.

8. Katheter mit Ballon nach Anspruch 7, dadurch gekennzeichnet, daß er einen Schaft, der in das Innenrohr eingesetzt werden kann, und auf dem Scheitel des Innenrohrs einen Stopper für den Schaft aufweist.

9. Katheter mit Ballon (2) mit dreischichtiger Struktur, die aus einem äußeren elastischen Film, einem inneren elastischen Film und einem dazwischenliegenden zylindrischen Textilerzeugnis aus Verbundgarn besteht, dadurch gekennzeichnet, daß das Verbundgarn ein Kern/Hüllen-Garn ist, wobei das Kerngarn elastisches Garn und das Hüllengarn nicht elastisches Garn ist, und wobei das Verhältnis der freien Länge des elastischen Garns zum nicht elastischen Garn 0,15 bis 0,5 beträgt, und daß das zylindrische Textilerzeugnis des Verbundgarns am äußeren Film und nicht am inneren Film haftet.

## Revendications

1. Cathéter à ballonnet (2) renforcé d'un fil composite composé d'un fil élastique et d'un fil non élastique d'une largeur libre supérieure à celle du fil élastique, caractérisé en ce que le fil composite est un fil à âme et gaine dans lequel le fil élastique est le fil d'âme et le fil non élastique est la gaine et dans lequel le rapport de longueur libre du fil élastique au fil non élastique est compris entre 0,15 et 0,5.

2. Cathéter à ballonnet selon la revendication 1, caractérisé en ce que le rapport de longueur libre du fil élastique au fil non élastique est compris entre 0,2 et 0,35.

3. Cathéter à ballonnet selon la revendication 1, caractérisé en ce que le fil à âme et gaine est un fil à âme et gaine dans lequel un fil retors de fibre synthétique est enroulé en spirale sur un fil d'uréthanne.

4. Cathéter à ballonnet selon la revendication 1, caractérisé en ce que le fil non élastique est un fil texturé à fausse torsion.

5. Cathéter à ballonnet selon la revendication 1, caractérisé en ce que le fil composite est tricoté ou tissé pour former une étoffe.

6. Cathéter à ballonnet selon la revendication 7, caractérisé en ce que l'étoffe est tricotée.

7. Cathéter à ballonnet selon la revendication 1, caractérisé en ce qu'une première extrémité du ballonnet est fixée sur le tube intérieur d'un double tube, dans lequel lesdits tubes peuvent coulisser concentriquement l'un par rapport à l'autre, et son autre extrémité est fixée sur le tube extérieur.

8. Cathéter à ballonnet selon la revendication 7, caractérisé en ce qu'il comprend une tige pouvant être insérée dans ledit tube intérieur et une butée pour ladite tige sur la pointe dudit tube intérieur.

9. Cathéter à ballonnet (2) comportant une structure à trois couches constituée d'un film élastique extérieur, d'un film élastique intérieur et d'une étoffe cylindrique de fil composite entre ceux-ci, caractérisé en ce que le fil composite est un fil à âme et gaine, dans lequel le fil d'âme est un fil élastique et le fil de gaine est un fil non élastique et dans lequel le rapport de longueur libre du fil élastique au fil non élastique est compris entre 0,15 et 0,5 et en ce que l'étoffe cylindrique de fil composite est collée au film extérieur et non au film intérieur.
